(19) Europäisches Patentamt
European Patent Office
Office européen des brevets

(11) Publication number: **0 494 648 A2**

(12) # EUROPEAN PATENT APPLICATION

(21) Application number: **92100162.4**

(51) Int. Cl.5: **A61L 31/00**, A61B 17/06

(22) Date of filing: **07.01.92**

(30) Priority: **07.01.91 US 638169**

(43) Date of publication of application:
**15.07.92 Bulletin 92/29**

(84) Designated Contracting States:
**BE CH DE ES FR GB IT LI LU NL SE**

(71) Applicant: **UNITED STATES SURGICAL CORPORATION**
**150 Glover Avenue**
**Norwalk, Connecticut 06856(US)**

(72) Inventor: **Granger, Richard N.**
**18 Arrowhead Lane**
**Huntington, CT 06484(US)**
Inventor: **Muth, Ross R.**
**97 Clearview Drive**
**Brookfield, CT 06804(US)**
Inventor: **Proto, George R.**
**31 Hillcrest Avenue**
**West Haven, CT 06516(US)**

(74) Representative: **Marsh, Roy David et al**
**Hoffmann Eitle & Partner Patent- und Rechtsanwälte Arabellastrasse 4 Postfach 81 04 20**
**W-8000 München 81(DE)**

(54) **Siliconized surgical needle and method for its manufacture.**

(57) A siliconized surgical needle is provided which requires significantly less force to effect tissue penetration than a standard siliconized needle. The silicone coating derives from an aminoalkyl siloxane.

EP 0 494 648 A2

## BACKGROUND OF THE INVENTION

This invention relates to a surgical needle possessing a silicone resin coating providing reduced tissue penetration force and to a method for manufacturing the needle.

The siliconization of metallic cutting edges of such articles as razor blades, hypodermic needles, scissors, scalpels and currettes has been known for some time.

U.S. Patent No. 3,574,673 discloses the silicone coating of a cutting edge employing a siliconization fluid containing a mixture of copolymerizable silicones made up of an aminoalkyl siloxane, specifically a (polyaminoalkyl) alkoxysilane, and a dimethylpolysiloxane.

Dow Corning Corporation's Bulletin 51-599A (July 1986) describes Dow Corning® MDX4-4159 Fluid for siliconizing cutting edges such as those previously mentioned with an ambient temperature and humidity-curable mixture of aminoalkyl siloxane and a cyclosiloxane dissolved in a mixture of Stoddard solvent and isopropyl alcohol. It is recommended that the fluid be applied by dipping, wiping, spraying, etc., in the form of a dilute organic solution, 2 e.g., prepared with a solvent such as hexane, trichlorotrifluoroethane, 1,1,1-trichloroethane or mineral spirits.

U.S. Patent No. 4,720,521 describes a film-forming siloxane composition for application to the aforementioned cutting edge articles which contains a mixture of three reactive siloxanes together with a non-reactive lubricating siloxane polymer.

## SUMMARY OF THE INVENTION

It is an object of the invention to provide a siliconized surgical needle and method for siliconizing a surgical needle in which the needle exhibits an average tissue penetration force below that of a standard siliconized surgical needle.

It is a particular object of the invention to provide a surgical needle with an adherent silicone coating derived from a siliconization material comprising an aminoalkyl siloxane and at least one other siloxane such as a cyclosiloxane which is copolymerizable therewith.

It is another particular object of the invention to provide a siliconization method to be carried out upon a surgical needle possessing an axial bore, or recess, for receiving the tip of a suture, the siliconization method omitting the step of occluding the bore with water as a preliminary to the application of the siliconization material to the needle.

In keeping with these and other objects of the invention, there is provided a siliconized surgical needle exhibiting an average tissue penetration force which is less than the average tissue penetration force of a standard siliconized needle.

A siliconized needle in accordance with this invention can be obtained by applying to a surface of the needle a siliconization material comprising an aminoalkyl siloxane and at least one other silicone copolymerizable therewith and thereafter curing the siliconization material to provide an adherent silicone coating on the needle.

The expression "standard siliconized surgical needle" as used herein refers to a commercially available siliconized surgical needle, e.g., the siliconized surgical needles marketed by Ethicon, Inc., Somerville, New Jersey.

While the amount of force required to achieve penetration of tissue during suturing may initially be about the same for both the siliconized surgical needle of this invention and a standard siliconized surgical needle and while both needles will tend to experience an increase in penetration force with each successive passage through tissue, at the conclusion of any given number of such passages, the needle of this invention will exhibit significantly less penetration force than the standard needle. Stated another way, the siliconized needle of this invention will retain its initial tissue penetration characteristics to a greater extent than a standard siliconized needle. This reduced tissue penetration force is advantageous inasmuch as it reduces the effort required in the suturing operation, a particular benefit in those cases involving extensive wound closure.

## DESCRIPTION OF THE PREFERRED EMBODIMENTS

The surgical needles which can be siliconized in accordance with this invention can be manufactured from a variety of metals such as Series 400 and Series 300 stainless steels. Other suitable metals for the fabrication of surgical needles include the quaternary alloys disclosed in U.S. Patent Nos. 3,767,385 and 3,816,920, the contents of which are incorporated by reference herein. A particularly preferred quaternary alloy possesses the ranges of components set forth in Table I as follows:

TABLE I

| COMPOSITION OF SURGICAL NEEDLE QUATERNARY ALLOY (WT.%) | | | |
|---|---|---|---|
| Component | Broad Range | Preferred Range | Most Preferred Range |
| Nickel | 10-50 | 24-45 | 30-40 |
| Cobalt | 10-50 | 25-45 | 30-40 |
| Nickel + Cobalt | 50-85 | 60-80 | 65-75 |
| Chromium | 10-30 | 12-24 | 15-22 |
| Molybdenum, tungsten and/or niobium (columbium) | 5-20 | 8-16 | 10-13 |

A particular quaternary alloy within Table I which can be utilized for the siliconized needle of this invention, designated MP35N, is available in wire form from Maryland Specialty Wire, Inc., Cockeysville, Maryland and contains (nominal analysis by weight): nickel, 35%; cobalt, 35%; chromium, 20% and molybdenum, 10%.

The siliconization material employed herein and the procedure used in its application will be such as to provide a siliconized surgical needle exhibiting a significantly reduced tissue penetration force compared with that of a standard surgical needle after an equivalent number of passages through the same, or substantially the same, tissue. Advantageously, the average tissue penetration force of the siliconized needle herein will be less than about 10 percent, preferably less than about 20 percent and still more preferably less than about 30 percent, of the average tissue penetration force of a standard siliconized needle after from 5 to 20 passes through the same or similar tissue.

In general, application of a curable siliconization material containing an aminoalkyl siloxane and at least one other copolymerizable siloxane, e.g., an alkyl polysiloxane or a cyclosiloxane, to a surgical needle followed by curing will provide a siliconized surgical needle meeting the requirements of this invention.

One suitable method for achieving siliconization herein utilizes the siliconization material and procedures described in U.S. Patent No. 3,574,673, the contents of which are incorporated by reference herein. The siliconization material includes (a) from about 5-20 weight percent of an aminoalkyl siloxane of the formula

$$Q_2N(CH_2)_3\overset{\displaystyle R}{\overset{|}{Si}}Y_bO_{\frac{3-a-b}{2}} \qquad \text{I}$$

in which R is a lower alkyl radical containing no more than about 6 carbon atoms; Y is selected from the group consisting of -OH and -OR' radicals in which R' is an alkyl radical of no more than 3 carbon atoms; Q is Selected from the group consisting of hydrogen, $-CH_3$ and $-CH_2CH_2NH_2$; a has a value of 0 or 1, and b has a value of 0 or 1 and the sum of a+b has a value of 0, 1 or 2 , and (b) from about 80 to 95 weight percent of a methyl substituted siloxane of the formula

$$R''\underset{\displaystyle CH_3}{\underset{|}{Si}}O_{\frac{3-c}{2}} \qquad \text{II}$$

in which R'' is selected from the group consisting of -OH and $-CH_3$ radicals and c has a value of 1 or 2.

In addition to, or in lieu of, the foregoing second copolymerizable siloxane, one can use one or more cyclosiloxanes, e.g., as described in the "Encyclopedia of Polymer Science and Engineering", Mark et al., eds., 2nd ed., John Wiley & Son (1989), Vol. 15, p. 207 et seq., the contents of which are incorporated by reference herein, provided, of course, the total amount of second copolymerizable siloxane(s) is within the aforestated range.

A particularly preferred siliconization material for use herein is Dow Corning Corporation's Dow Corning® MDX 4-4159 Fluid ("MDX Fluid"), a 50 percent active solution of dimethyl cyclosiloxanes and dimethoxysilyldimethylaminoethylaminopropyl silicone polymer in a mixture of Stoddard solvent (mineral

spirits) and isopropyl alcohol. MDX Fluid can be applied to a surface of the cleaned surgical needle by dipping, wiping, spraying, etc., in the form of a dilute organic solution, e.g., prepared with a solvent such as hexane, trichlorotrifluoroethane, 1,1,1-trichloroethane or mineral spirits. In general, it is preferred to dilute MDX Fluid (or other siliconization material) in a hydrocarbon solvent possessing from 5 to 10 carbon atoms, e.g., pentane, hexane (which is preferred), heptane, octane, etc. MDX Fluid cures at room temperature to provide an adherent silicone coating.

After evaporation of any diluent or solvent carrier, the siliconization material is cured to the desired degree. The material can be cured by heating for a short time, e.g., 30 minutes at 120 ° C, or by exposure to ambient temperature and humidity conditions for longer periods of time.

As previously mentioned, where an axially bored surgical needle is concerned, it is preferred to siliconize the needle employing a procedure which does not require the preliminary step of temporarily occluding the bore. Typically, when siliconizing such a needle by dipping or total immersion in the siliconization material, it has been found necessary to occlude the bore with a liquid, e.g., water, which is immiscible with the siliconization material and thus prevents any of such material from entering the bore where it might interfere with proper attachment of the suture. It has been found that the bore-occluding step can be totally omitted by applying the siliconization material to the needle by spraying. Accordingly, spraying is a preferred method of application of the siliconization material at least in the case of a needle possessing an axial bore, or recess.

Spraying is also the preferred method for applying siliconization fluid to a needle possessing a reduced shank end which is intended to be attached to the tip of a suture employing a shrinkable tubular connector as disclosed in commonly assigned copending U.S. patent application Serial No. 07/413,240, filed September 27, 1989, the contents of which are incorporated by reference herein. If is preferred in the case of such a needle to insert the needle shank end - first into a support block, e.g., of rigid foam, and thereafter to spray the siliconization fluid onto the exposed surface of the needle. Since the shank end of the needle is embedded in the support block, it will remain free of silicone during the spraying procedure. The use of a support block can, of course, also be employed in the case of the axial recess type needle described above to prevent siliconization material from entering the recess. It is preferable that the coated needle while still in its support block be subjected to curing conditions; if this involves heat, it will, of course, be necessary to select a support block material which can withstand the elevated temperature selected for curing.

The following examples are illustrative of the siliconized surgical needle of this invention and the method for its manufacture.

## EXAMPLE 1

This example illustrates the coating of a quantity of surgical needles made from .039 inch diameter surgical grade stainless steel wire configured as a $\frac{1}{2}$ circle curved taper point general surgical needle having a length of 37 millimeters (Needle A). Each needle possessed an axial recess at its blend end for receiving the tip of a suture.

The needles were placed in a basket and immersed in an ultrasonic cleansing unit for 5 minutes. The basket was raised to the vapor section of the unit and held there for another 5 minutes. The needles were then dried and after 20 minutes were transferred to a second basket which was immersed for 30 seconds in a siliconization medium prepared from 1 part by volume of MDX Fluid and 9 parts by volume of hexane as solvent. Following drainage of excess siliconization medium, the needles were spread on a tray and heated for 16 hours at 120 ° C to effect curing of the silicone coating.

## EXAMPLE 2

This example compares the penetration characteristics of Needle A of Example 1 with a commercial siliconized surgical needle of the same diameter and configuration, specifically, Ethicon Inc.'s CT-1 surgical needle (Needle B).

Needle A was tested by passing 78 samples of the needle through a Porvair (Inmont Corporation), a microporous polyurethane membrane of about .042 inches thickness which served to simulate flesh. The amount of force in grams to achieve penetration of the Porvair by the needle was measured for each of ten successive penetrations for each of the 78 needle samples. Measurement of the needle penetration force was accomplished using the test procedure and apparatus described in commonly assigned copending U.S. patent application Serial No. 07/541,055, filed June 20, 1990, the contents of which are incorporated by reference herein. The test was performed by a testing fixture and an Instron Universal Testing Machine. The surgical needles were mounted in a gripping clamp which fixed the needle in a position perpendicular to the

Porvair surface and oriented on its radial profile with the axis of rotation on the same plane as the plane of the Porvair. The needle was rotated into the Porvair which was mounted on top of an Instron load cell. The maximum amount of vertical force is recorded as the needle is pushed through the Porvair.

Needle B was tested in the same way as Needle A except that 73 individual needle samples were evaluated.

The average penetration force for all needles measured with each successive passage through Porvair and the average penetration force of the needles after all ten passages through Porvair are set forth in Table I as follows:

TABLE I

| Needle Penetration Force Average Penetration Force (gm) | | | | | | | | | | | |
|---|---|---|---|---|---|---|---|---|---|---|---|
| Passage Through Porvair | 1 | 2 | 3 | 4 | 5 | 6 | 7 | 8 | 9 | 10 | Average of 10 Successful Passages |
| Needle A | 196 | 278 | 326 | 373 | 396 | 411 | 429 | 450 | 477 | 484 | 381 |
| Needle B | 207 | 284 | 393 | 490 | 603 | 657 | 681 | 732 | 747 | 791 | 557 |

As these data show, although the average penetration force of both sets of Needles A and B was about the same upon the first passage of the needles through Porvair, and with each successive passage, greater force was required to achieve penetration, from the third penetration to the last, the tenth, penetration, Needle A required less force to effect penetration than Needle B and the average penetration force for all ten passes through Porvair in the case of Needle A was 30% less than that required for Needle B.

EXAMPLE 3

This example illustrates the siliconization of 10 samples of Needles A of Example 1 employing a spraying procedure. Prior to spraying, the needles were ultrasonically cleaned as in Example 1 and transferred to a tray where they laid on their sides. The siliconization fluid of Example 1 was sprayed onto the needles employing a spray bottle and the fluid was allowed to spread evenly over the needles' surfaces for a period of about 30 minutes. thereafter, the needles were baked to cure the siliconization fluid. Unlike the siliconization method employed in Example 1 where siliconization fluid tended to migrate into the axial recess formed in the blunt end of the needle, there was a much reduced tendency of the fluid to enter the recess when applied by the spraying procedure of this example. Thus, spraying appears to be a more advantageous technique for applying siliconization material to the needle so as it tends to minimize or avoid the presence of silicone in the needle recess, a material which might interfere with proper suture attachment.

Employing the needle penetration force testing procedure described in Example 2, the following penetration data were obtained:

TABLE II

| Needle Penetration Force Average Penetration Force (gm) | | | | | | | | | | | |
|---|---|---|---|---|---|---|---|---|---|---|---|
| Passage Through Porvair | 1 | 2 | 3 | 4 | 5 | 6 | 7 | 8 | 9 | 10 | Average of 10 Successful Passages |
| Needle A | 143 | 154 | 181 | 215 | 253 | 289 | 312 | 328 | 358 | 417 | 260 |

**Claims**

1. A method for manufacturing a siliconized surgical needle which comprises applying to a surface of the needle a siliconization material comprising an aminoalkyl siloxane and curing the siloxane thereon to provide a silicone coating.

2. A method for manufacturing a siliconized surgical needle from a surgical needle possessing a suture

top-receiving axial recess in its blunt end which comprises spraying a curable siliconization material upon the needle while its recess is unoccluded, there being no significant amount of the siliconization material entering the bore, and thereafter curing the siliconization material on the needle to provide a silicone coating thereon.

**3.** A method according to claim 2, wherein the shank end of the needle is embedded in a support material during spraying.

**4.** A method for manufacturing a siliconized surgical needle from a surgical needle possessing a shank of reduced diameter which comprises embedding the shank in a support material, spraying a curable siliconization material upon the exposed surface of the needle and thereafter curing the siliconization material on the needle to provide a silicone coating thereon.

**5.** A method according to claim 1, 2, 3 or 4 wherein the silicone coating on the needle derives from a siliconization material comprising an aminoalkyl siloxane and at least one other siloxane.

**6.** A method according to claim 5, wherein the siliconization material includes a cyclosiloxane.

**7.** A method according to claim 6 wherein the siliconization material includes at least one hydrocarbon solvent of from 5 to 10 carbon atoms.

**8.** A method according to claim 7 wherein the hydrocarbon solvent is selected from hexane and heptane.

**9.** A method according to any one of the preceding claims wherein the needle is fabricated from an alloy comprising nickel, cobalt, chromium and at least one metal selected from molybdenum, tungsten and niobium.

**10.** A method according to claim 9, wherein the alloy comprises from about 10 to about 50 weight percent nickel, from about 10 to about 50 weight percent cobalt with the combined weight of nickel and cobalt being from about 50 to about 85 weight percent, from about 10 to about 30 weight percent chromium and from about 5 to about 20 weight percent of said at least one metal selected from molybdenum, tungsten and niobium.

**11.** A siliconized surgical needle which exhibits an average tissue penetration force which is less than the average tissue penetration force of a standard siliconized surgical needle.

**12.** A needle as claimed in claim 11 which exhibits an average tissue penetration force which is at least 10% less than the average tissue penetration force of a standard siliconized surgical needle after an equivalent number of consecutive passes through the same, or substantially the same, tissue.

**13.** A needle as claimed in claim 12, which exhibits an average tissue penetration force which is at least 20% less than the average tissue penetration force of a standard siliconized surgical needle after an equivalent number of consecutive passes through the same, or substantially the same, tissue.

**14.** A needle as claimed in claim 13, which exhibits an average tissue penetration force which is at least 30% less than the average tissue penetration force of a standard siliconized surgical needle after an equivalent number of consecutive passes through the same, or substantially the same, tissue.

**15.** A needle as claimed in any one of claims 11 to 14, which exhibits an average tissue penetration force after 10 successive passages of less than about 500 grams.

**16.** A needle as claimed in any one of claims 11 to 15 wherein the needle possesses a diameter beneath the coating of about 1mm (.039 inches).

**17.** A needle as claimed in any one of claims 11 to 16, and fabricated from an alloy comprising from about 10 to about 50 weight percent nickel, from about 10 to about 50 weight percent cobalt with the combined weight of nickel and cobalt being from about 50 to about 85 weight percent, from about 10 to about 30 weight percent chromium and from about 5 to about 20 weight percent of at least one metal

selected from molybdenum, tungsten and niobium.

**18.** A method of applying a silicone coating to a surgical needle which has an axial bore or recess for receiving the tip of a suture the method being characterised by omission of any step of occluding the bore or recess with water prior to application of siliconization coating material to the needle.

**19.** A method according to claim 18 wherein the coating is applied by a spraying step.